(19) Europäisches Patentamt
European Patent Office
Office européen des brevets

(11) Veröffentlichungsnummer: **0 000 764**
**B1**

(12) # EUROPÄISCHE PATENTSCHRIFT

(45) Veröffentlichungstag der Patentschrift: **05.05.82**

(51) Int. Cl.³: **C 07 C 103/52,** A 61 K 37/02

(21) Anmeldenummer: **78100564.0**

(22) Anmeldetag: **01.08.78**

(54) **Antikonzeptionsmittel enthaltend ein Peptid.**

(30) Priorität: **06.08.77 DE 2735515**

(43) Veröffentlichungstag der Anmeldung:
**21.02.79 Patentblatt 79/4**

(45) Bekanntmachung des Hinweises auf die Patenterteilung:
**05.05.82 Patentblatt 82/18**

(84) Benannte Vertragsstaaten:
**BE CH DE FR GB NL**

(56) Entgegenhaltungen:
**FR-A-2281132**
**FR-A-2348913**
**US-A-3914412**

**CHEMICAL ABSTRACTS,**
**B. 85-réference No. 186906 h (1976)**

**JOURNAL OF MEDICINAL CHEMISTRY,**
**(Ausg. American Chemical Society)**
**B. 19, 1976, Seiten 243–45.**

(73) Patentinhaber: **HOECHST AKTIENGESELLSCHAFT, Postfach 80 03 20, D-6230 Frankfurt/Main 80 (DE)**

(72) Erfinder: **von der Ohe, Marianne, Dr., Biebricher Allee 112, D-6200 Wiesbaden (DE)**
Erfinder: **Sandow, Jürgen Kurt, Dr., Am Haidplacken 22, D-6240 Königstein/Taunus (DE)**
Erfinder: **von Rechenberg, Wolfrad, Dr., Taunusstrasse 76, D-6233 Kelkheim (Taunus) (DE)**

**EP 0 000 764 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Antikonzeptionsmittel enthaltend ein Peptid

Gegenstand der Erfindung ist ein Antikonzeptionsmittel auf der Grundlage eines Peptids der allgemeinen Formel

$$\ulcorner Glu-His-Trp-Ser-Tyr-X-Leu-Arg-Pro-NHC_2H_5 \qquad (I)$$

in der X für D-Serin-tert-butyläther steht.

Die Verbindung der Formel I wird gemäss DOS 2 438 350 hergestellt. Sie bewirkt nach einmaliger Gabe in Dosierungen von 1.4–20 mcg (das entspricht 20–300 ng/kg) intravenös (i. v.) 50–1000 mcg (das entspricht 100–5000 ng/kg) intranasal (i. n.) bei Versuchspersonen die Ausschüttung von Gonadotropinen aus den Hypophysen-Vorderlappen.

Es wurde nun gefunden, dass bei wiederholter täglicher Dosierung über mindestens 4 Tage von mehr als 2,5 mcg i. v., s. c. oder i. m. (Tagesdosis) bzw. mehr als etwa 100 mcg i. n. die Wirkung sich nicht verstärkt, sondern überraschenderweise umkehrt, so dass bei entsprechender Dosierung diese Verbindung als Antikonzeptivum verwendet werden kann.

Die Dosierung beträgt z. B. täglich 3 × 5 mcg s. c., i. v. oder i. m., kann aber unbedenklich auf das Vielfache gesteigert werden, da das Peptid untoxisch ist. In praxi bewegt man sich zwischen 5–500 mcg täglich. Bei intranasaler Anwendung muss diese Dosis wegen der Resorption von nur ca. 1% auf das etwa Hundertfache angehoben werden.

Das erfindungsgemässe Mittel kann beim Mann zur temporären Unterdrückung der Testosteronproduktion und damit Spermatogenese verwendet werden, bei der Frau zur Unterdrückung der Ovulation. Die Applikation bei der Frau beginnt z. B. am ersten Zyklustag und wird jeweils mindestens 14 Tage fortgesetzt. Auf diese Weise wird die Ovulation während des Behandlungszeitraumes mit Sicherheit verhindert. Der LH-Spiegel sinkt auf Basalwerte, und eine Stimulierung der Hypophyse ist nicht mehr möglich. Bei kurzzeitiger Behandlung wird sie unterdrückt. Letzteres gilt ggf. auch für die Nidation, da beide Vorgänge von der Höhe des FSH- und LH-Spiegels abhängen.

Bisher war versucht worden, die LH- und FSH-Ausschüttung durch Anwendung kompetitiver Hemmer des LH-RH zu unterdrücken. Das gelang aber nur unzureichend und mit Konzentrationen, die um das etwa Tausendfache höher liegen als die der erfindungsgemässen Verbindung.

Zudem entfallen die z. B. bei steroidhaltigen Kontrazeptiva zu berücksichtigenden Rückstands- und Metabolismusprobleme. Die erfindungsgemässen Zubereitungen enthalten Peptide, die im Körper leicht zu Aminosäuren abgebaut und ausgeschieden oder über diese Aminosäuren in physiologischer Weise metabolisiert werden.

Verbindung der Formel I kann zur Verwendung z. B. in physiologischer Kochsalzlösung intravenös, intramuskulär oder subkutan appliziert werden. Als medizinisch besonders wertvolle Anwendungsform eignen sich insbesondere für Dauerbehandlung wässrige oder ölige Zubereitungen zur intranasalen Applikation. In Form einer Zubereitung als Zäpfchen ist auch rektale oder vaginale Anwendung möglich.

a) Beobachtung an Männern

3 Männer im Alter von 30 – 55 Jahren erhielten 10 Tage lang täglich 50 mcg Verbindung I subkutan in wässriger Zubereitung. Die Testosteronspiegel wurden durch Radioimmunoassay gemessen. Es fand sich eine Senkung der Testosteronspiegel im Plasma auf ca. ⅓ des Ausgangswertes. 6 Wochen nach Absetzen der Behandlung wurden die Testosteronspiegel erneut gemessen. Sie hatten wieder ihren normalen Wert erreicht.

Dieselben Ergebnisse wurden erhalten, wenn 10 Tage lang täglich 2 mg in öliger Zubereitung intranasal verabreicht wurden.

b) Beobachtung an Frauen

4 Frauen im Alter von 22 – 30 Jahren erhielten täglich 3 × 5 mcg Verbindung I subkutan in wässriger Zubereitung ab Zyklusbeginn. Wenige Tage nach Behandlung waren die Plasmaspiegel an luteinisierenden und follikelstimulierendem Hormon auf Basalwerte abgesunken und eine Stimulierung der Hypophyse trat nicht mehr ein.

Bei kurzzeitiger Behandlung wurde die Ovlation verschoben, während sie bei längerer Behandlung ganz unterdrückt wurde.

c) Beobachtung an Tieren

2 Gruppen von je 5 geschlechtsreifen, reinrassigen Beagle-Rüden, Gewicht 6–8 kg, erhielten 1 × täglich 2.5 mcg/kg Verbindung I in isotonischer Kochsalzlösung subkutan verabreicht. Die Testosteronspiegel sanken bereits nach 1 Monat auf 30% des Ausgangswerts, nach 3 Monaten auf 6% des Ausgangswertes ab.

Die Behandlung wurde 6 Monate fortgesetzt. Im Verlauf der Behandlung trat deutliche Hodenatropie auf, jedoch stieg 8 Wochen nach Absetzen der Behandlung die Hodengrösse wieder deutlich an, und die Tiere zeigten einen normalen Geschlechtstrieb.

In den folgenden Beispielen wird die Zubereitung entsprechender Präparate beschrieben.

Beipiel 1

500 mg Verbindung I werden in 100 Liter physiologischer Kochsalzlösung gelöst. Man filtriert steril und füllt in Ampullen zu je 1 ml ab. Diese Zubereitung kann zur intravenösen, intramuskulären oder subkutanen Verabreichung der Verbindung I dienen.

Beispiel 2

600 mg Verbindung I werden in 100 l isotonischer wässriger Mannitlösung gelöst und wie in Beipiel 1 weiterbehandelt. Die ampullierte Lösung wird gefriergetrocknet zum Gebrauch wieder in 1 ml dest. Wasser gelöst.

Beispiel 3

9 l dest. Wasser werden zum Sieden erhitzt und darin 20 g 4-Hydroxybenzoesäure-methylester gelöst. Man kühlt auf ca. 30° C, gibt 89,6 g $Na_2NPO_4$, 13.5 g Citronensäure, 10 g Natriumchlorid und 250 g Mannit zu und löst dann darin 100 g Verbindung I. Dann füllt man mit dest. Wasser auf 10 l auf und filtriert.

1 ml Lösung wird z. B. in einen Behälter gefüllt, der mit einem Entnahme-Dosierventil versehen ist, das pro Applikation 0.05 ml der Lösung freigibt.

Beispiel 4

10 g Benzylalkohol werden mit 2-Octyldodekanol auf 0.990 l aufgefüllt. Dazu gibt man 10 mg mikrofein gemahlene Verbindung I und homogenisiert.

1–2 ml dieser Suspension werden in kleine Behälter abgefüllt. Die Behälter sind mit einem Entnahmeventil versehen, das pro Applikation 0.05 ml der Suspension freigibt.

Beipiel 5

2 kg handelsüblicher Suppositorienmassen wird auf ca. 60° C erwärmt. Man versetzt mit 2 g mikrofein gemahlener Verbindung I und homogenisiert. Die Masse wird in Formen gegossen, die nach dem Erkalten Suppositorien im Gewicht von jeweils 2 g freigeben.

**Patentanspruch**

Antikonzeptionsmittel, enthaltend ein Peptid der Formel I

Glu–His–Trp–Ser–Tyr–X–Leu–Arg–Pro–$NHC_2H_5$

in der X für D-Serin-tert-butyläther steht, für intravenöse, subkutane, intramuskuläre oder intranasale Applikation, gekennzeichnet durch die Konfektionierung in Dosen für mindestens vier Tage, wobei jede Tagesdosis mehr als 2.5 mcg für intravenöse, subkutane oder intramuskuläre Applikation bzw. mehr als 100 mcg für intranasale Applikation ausmacht.

**Claim**

A contraceptive drug containing a peptide of the formula I

Glu–His–Trp–Ser–Tyr–X–Leu–Arg–Pro–$NHC_2H_5$

in which X stands for D-serin-tert. butyl ether for intraveneous, subcutaneous, intramuscular or intranasal administration, characterized by dosage units for at least 4 days wherein each dayly dosage contains more than 2.5 micrograms for intraveneous, subcutaneous or intramuscular administration and more than 100 micrograms for intranasal administration, respectively.

**Revendication**

Agent anticonceptionnel contenant un peptide de formule I

Glu–His–Trp–Ser–Tyr–X–Leu–Arg–Pro–$NHC_2H_5$

dans laquelle X désigne l'éther tert-butylique de la D-sérine, pour l'administration intraveineuse, sous-cutanée, intramusculaire ou intranasale, caractérisé en ce qu'il est mis sous forme de doses pour au moins 4 jours, chaque dose quotidienne représentant plus de 2,5 mcg pour l'administration intraveineuse, sous-cutanée ou intramusculaire, ou plus de 100 mcg pour l'administration intranasale.